# EUROPÄISCHE PATENTSCHRIFT

(11) **EP 0 316 650 B1**
(45) Veröffentlichungstag und Bekanntmachung des Hinweises auf die Patenterteilung: **12.01.1994**
(21) Anmeldenummer: 88118182.0
(22) Anmeldetag: 02.11.1988
(51) Int. Cl.: C07K 1/14, C07K 7/10, A61K 35/62

(54) **Verfahren zur Isolierung und Reinigung von Hirudin**
Process for the separation and purification of hirudin
Procédé de séparation et de purification de la hirudine

(30) Priorität: 13.11.1987 DE 3738541
(43) Veröffentlichungstag der Anmeldung: 24.05.1989
(73) Patentinhaber: HOECHST AKTIENGESELLSCHAFT, 65926 Frankfurt am Main (DE)
(72) Erfinder: Badziong, Werner, Dr., D-6232 Bad Soden am Taunus (DE); Crause, Peter, Dr., D-6050 Offenbach (DE); Habermann, Paul, Dr., D-6239 Eppstein/Taunus (DE); Tripier, Dominique, Dr., D-6239 Eppstein/Taunus (DE)

(56) Entgegenhaltungen:
- EP-A- 0 158 986
- EP-A- 0 171 024
- EP-A- 0 209 061

## Beschreibung

Die Erfindung betrifft ein Verfahren zur Isolierung und Reinigung von Hirudin aus komplexen Kulturfiltraten und salzhaltigen Lösungen.

Bei dem ursprünglich aus dem Blutegel Hirudo medicinalis isolierten Polypeptid Hirudin handelt es sich um einen hochspezifischen Thrombininhibitor mit breitem therapeutischen Potential (F. Markwardt, Biomed. Biochim. Acta 44 (1985) 1007 - 1013). Die benötigten Mengen können jedoch nur auf gentechnologischem Wege über transformierte Mikroorganismen hergestellt werden. Dabei hat es sich gezeigt, daß die Hefe Saccharomyces cerevisiae als Wirtsorganismus geeignet ist, um korrekt gefaltetes und voll aktives Hirudin zu produzieren (EP A1 168 342, EP A1 200 655). Die Sezernierung des Proteins ergibt Konzentrationen von bis zu einigen hundert Milligram Hirudin pro Liter Kulturfiltrat. Allerdings ist eine hohe Ausbeute des Proteins nur dann zu erzielen, wenn bei der Hefefermentation komplexe Nährmedien unter Zusatz von Hefeextrakt, Cornsteep, Pepton oder Fleischpaste eingesetzt werden, so daß sich für die Reinigung des Proteins das Problem stellt, Hirudin aus hoher Verdünnung in einem Gemisch von proteinartigen Begleitstoffen zu isolieren. Die Ionenaustauschchromatographie, wie sie für die Isolierung des Hirudins aus Egelextrakten beschrieben ist (P. Walsmann and F. Markwardt, Thromb. Res. 40 (1985) 563-570), scheidet aufgrund des hohen Salzgehalts der verwendeten Nährlösungen als primärer Schritt aus. Auch das gewöhnlich praktizierte Verfahren der Ultrafiltration zur Entsalzung und Konzentrierung der Proteinlösung hat erhebliche Nachteile. So werden teure apparative Ausstattungen benötigt, der Massenproduktion durch die lange Prozeßdauer Grenzen gesetzt und durch die Mitanreicherung die Tendenz zur Präzipitation von Inhaltsstoffen des Nährmediums gefördert, woraus wiederum technische Schwierigkeiten resultieren.

Aus EP A2 049 847 ist ein Verfahren zur Reinigung des α-Amylaseinhibitors aus Kulturfiltraten von Streptomyces tendae unter Verwendung von Adsorptionsharzen bekannt. Ein Verfahren zur Reinigung von Proinsulin durch ®Amberlite XAD-Harze unter Verwendung wäßriger Elutionsmittel mit einem Gehalt von 10 bis 30 % Aceton oder Acetonitril wird in EP A2 197 764 beschrieben.

Der Erfindung liegt die Aufgabe zugrunde, ein Verfahren zu entwickeln, mit dem es möglich ist, aus komplexen Hefekulturfiltraten Hirudin auf einfache Weise und in guter Ausbeute zu isolieren.

Diese Aufgabe wird erfindungsgemäß durch das Verfahren zur Isolierung und Reinigung von Hirudin aus komplexen und salzhaltigen Lösungen durch hydrophobe Chromatographie gelöst, dadurch gekennzeichnet, daß die hydrophobe Chromatographie an porösen Adsorberharzen mit einem Porendurchmesser von 50 bis 5000 Å und einer spezifischen Oberfläche von mindestens 50 m²/g unter Verwendung einer 10 bis 40 %igen Lösung eines oder mehrerer mit Wasser mischbarer organischer Lösungsmittel als Eluent durchgeführt wird.

Als poröse Adsorberharze werden bevorzugt Copolymere von Styrol und Divinylbenzol, wie z.B. ®Diaion HP 10, 20, 30, 40, 50, oder Copolymere von Acrylatester und Divinylbenzol, wie z.B. ®Amberlite XAD-7 und 8 (Fa. Röhm und Haas), insbesondere ®Diaion HP 20 und ®Amberlite XAD-7 verwendet. Als mit Wasser mischbare organische Lösungsmittel kommen z.B. Methanol, Ethanol, n-Propanol, Isopropanol und Aceton in Frage. Es wird bevorzugt eine Lösung eines mit Wasser mischbaren organischen Lösungsmittels verwendet.

Die Durchführung des erfindungsgemäßen Verfahrens kann sowohl in Form einer Batch- als auch einer Säulenchromatographie erfolgen. Das Harz wird mit einem wäßrigen Puffer oder einer Lösung einer organischen Säure, wie z.B. 0 bis 0,2 M Essigsäure, voräquilibriert. Die Hirudinhaltige Aufgabelösung, insbesondere Kulturfiltrat von Saccharomyces cerevisiae, wird auf einen pH von 2 bis 8, vorzugsweise 3 bis 5 eingestellt, und mit dem Harz in Kontakt gebracht. Nach vollständiger Bindung des Hirudins wird das Harz mit einem wäßrigen Puffer (pH 2 bis 9), z.B. Tris, Ethylendiamin, und/oder einer Lösung einer organischen Säure, z.B. 0 bis 0,2 M Essigsäure, gewaschen. Die Elution erfolgt dann mit Hilfe einer 10 bis 40 %igen Lösung eines mit Wasser mischbaren organischen Lösungsmittels. Der wäßrige Anteil des Eluenten kann aus einem wäßrigen Puffer, einer Lösung einer organischen Säure, wie z.B. Essigsäure, oder Wasser bestehen. Vorzugsweise werden jedoch Lösungen von 20 bis 30 % Isopropanol mit 0 bis 0,1 M Essigsäure eingesetzt, um salzfreie Eluate zu erhalten.

Das Verfahren ist in analoger Weise geeignet, Hirudin aus salzhaltigen Lösungen, beispielsweise Ionenaustauschereluaten, salzfrei zu erhalten.

Die Erfindung findet Anwendung für die Reinigung von rekombinanten Hirudinen, insbesondere solchen, die in Saccharomyces cerevisiae zur Expression gebracht werden. Unter Hirudinen sind peptidartige Thrombininhibitoren mit einer spezifischen Aktivität von mindestens 10000 AT-U/mg zu verstehen, die von den bekannten Isohirudinen der Spezies Hirudo medicinalis abgeleitet sind und wesentliche Strukturmerkmale dieser, insbesondere die charakteristische Verknüpfung der drei Disulfidbrücken (J. Dodt et al., Biol. Chem. Hoppe-Seyler 366 (1985) 379-385), aufweisen (vgl. z.B. EP A1 158 564, EP A1 168 342, DE 3445517 A1, EP A2 193 175, EP A1 200 655, EP A1 158 986, EP A1 209 061, DE 3342139, EP A1 171 024).

Insbesondere werden darunter Hirudine verstanden, wie sie in EP A1 171 024, EP A1 158 986 und EP A1 209 061 beschrieben sind.

Das erfindungsgemäße Verfahren zeichnet sich dadurch aus, daß Hirudin aus komplexen und salzhaltigen Lösungen, z.B. komplexen Hefekulturfiltraten, quantitativ an Adsorberharze gebunden werden kann, ohne daß eine Beeinträchtigung der Tertiärstruktur und Aktivität erfolgt. Durch Elution mit wäßrigen Lösungen unter Zusatz von mit Wasser mischbaren organischen Lösungsmitteln kann das Hirudin angereichert in einer salz- und trübstofffreien Lösung erhalten werden, die direkt, beispielsweise durch Ionenaustauschchromatographie, weiterverarbeitet werden kann.

Die nachfolgenden Beispiele sollen die Erfindung näher erläutern, jedoch ohne darauf einzuschränken:

### Beispiel 1

Man nimmt Kulturmedium aus der Fermentation eines Hefestammes der Spezies Saccharomyces cerevisiae, die dem Matα-mating-Type entspricht und mit Hefe-Hirudin-Expressionsplasmid in einem Analogverfahren zu Brake et al., PNAS, Vol. 81, (1984) 4632-4646 transformiert ist.

950 l Kulturfiltrat von Saccharomyces cerevisiae enthaltend 20 g/l Hefeextrakt und 12 mg/l Hirudin wurden über eine Säule von 100 l ®Diaion HP 20, äquilibriert mit 20 mM Essigsäure, gegeben. Nach der Aufgabe wurde mit 1000 l 50 mM Tris/HCl pH 8,5, gefolgt von 300 l 20 mM Essigsäure, nachgewaschen. Dann wurde mit 300 l 20 mM Essigsäure, enthaltend 20 % Isopropanol, gefolgt von 500 l 20 mM Essigsäure, enthaltend 30 % Isopropanol, eluiert. Die Eluate wurden fraktioniert und auf ihren Hirudingehalt analysiert. Drei Fraktionen (150 l) wurden vereinigt und enthielten 9,5 g Hirudin entsprechend einer Ausbeute von 84 %. Die essigsaure Lösung wurde dann mit 1 M Piperazin auf pH 6,0 eingestellt und mit 10 kg Matrex ®Cellufine AM, äquilibriert mit 20 mM Piperazin (pH 6,0), verrührt. Dabei wurde das Hirudin quantitativ an den Ionenaustauscher gebunden. Das Material wurde mit 20 mM Piperazin (pH 6,0) gewaschen und in eine Säule gefüllt. Durch Elution mit einem Salzgradienten von 0 bis 0,3 M NaCl im gleichen Puffer konnten 6,7 g Hirudin hochangereichert und konzentriert in 6 l Lösung erhalten werden.

### Beispiel 2

1,2 l eines Ionenaustauschereluats enthaltend 2,0 g Hirudin, 20 mM Piperazin (pH 6,0) und 200 mM NaCl wurden angesäuert auf 0,1 M Essigsäure und mit einer Flußrate von 400 ml/h über eine Säule von 100 ml ®Diaion HP 20 in 0,1 M Essigsäure gegeben. Nach erfolgter Probenaufgabe wurde das Harz mit 10 mM Essigsäure salzfrei gewaschen. Die Elution erfolgte dann bei einer Flußrate von 200 ml/h mit 30 % Isopropanol in 10 mM Essigsäure. Das Hirudin wurde in einem Gesamtvolumen von 200 ml aufgefangen. Nach Gefriertrocknung der Lösung verblieben 1,7 g salzfreies Hirudin.

### Beispiel 3

754 l Kulturfiltrat von Saccharomyces cerevisiae mit einem Gehalt von 27,2 g Hirudin wurden mit 4,9 l Essigsäure auf pH 4 gestellt und mit einer Suspension von 75 kg ®Diaion HP 20 in 75 l 30 %igem Isopropanol verrührt. Nach 30 Minuten wurde das Kulturfiltrat über eine Drucknutsche abfiltriert und verworfen. Das in der Nutsche verbleibende Adsorberharz wurde mit 700 l 20 mM Tris/HCl pH 8,5 und 250 l 0,1 M Essigsäure gewaschen. Dann wurde das Hirudin durch zehn aufeinanderfolgende Waschungen mit je 50 l 30 % Isopropanol in 20 mM Essigsäure eluiert. Die Eluate wurden auf ihren Hirudingehalt analysiert. Vier Fraktionen (195 l) wurden vereinigt und enthielten 22,6 g Hirudin entsprechend einer Ausbeute von 83 %.

## Patentansprüche

1. Verfahren zur Isolierung und Reinigung von Hirudin aus komplexen und salzhaltigen Lösungen durch hydrophobe Chromatographie, dadurch gekennzeichnet, daß die hydrophobe Chromatographie an porösen Adsorberharzen mit einem Porendurchmesser von 50 bis 5000 Å und einer spezifischen Oberfläche von mindestens 50 m²/g unter Verwendung einer 10 bis 40 %igen Lösung eines oder mehrerer mit Wasser mischbarer organischer Lösungsmittel als Eluent durchgeführt wird.

2. Verfahren gemäß Anspruch 1, dadurch gekennzeichnet, daß als poröse Adsorberharze Copolymere von Styrol und Divinylbenzol oder Copolymere von Acrylatester und Divinylbenzol eingesetzt werden.

3. Verfahren gemäß Anspruch 1 oder 2, dadurch gekennzeichnet, daß als mit Wasser mischbare organische Lösungsmittel Methanol, Ethanol, n-Propanol, Isopropanol und Aceton eingesetzt werden.

## Claims

1. A process for the isolation and purification of hirudin from complex and salt-containing solutions by hydrophobic chromatography, which comprises carrying out the hydrophobic chromatography on porous adsorber resins with a pore diameter of 50 to 5000 Å and a specific surface area of at least 50 m²/g, using as eluent a 10 to 40 % strength solution of one or more organic solvents which are miscible with water.

2. The process as claimed in claim 1, wherein copolymers of styrene and divinylbenzene, or copolymers of acrylate ester and divinylbenzene, are used as porous adsorber resins.

3. The process as claimed in claim 1 or 2, wherein methanol, ethanol, n-propanol, isopropanol and acetone are used as organic solvents which are miscible with water.

## Revendications

1. Procédé d'isolement et de purification de l'hirudine de solutions complexes contenant des sels par chromatographie hydrophobe, caractérisé en ce que la chromatographie hydrophobe s'effectue sur une résine adsorbante poreuse ayant une porosité de 50 à 5000 Å et une surface spécifique d'au moins 50 m²/g avec l'utilisation de 10 à 40 % d'une solution d'un ou de plusieurs solvants organiques miscibles à l'eau en tant qu'éluant.

2. Procédé selon la revendication 1, caractérisé en ce que la résine adsorbante poreuse est un copolymère de styrène et de divinylbenzène ou un copolymère d'ester acrylique et de divinylbenzène.

3. Procédé selon la revendication 1 ou 2, caractérisé en ce que le solvant organique miscible à l'eau est le méthanol, l'éthanol, le n-propanol, l'isopropanol et l'acétone.
